# EUROPEAN PATENT APPLICATION

(11) **EP 2 338 989 A1**
(43) Date of publication of application: **29.06.2011**
(21) Application number: 09809902.1
(22) Date of filing: 25.08.2009
(51) Int. Cl.: C12N 15/09, C12Q 1/68

(54) **METHOD FOR PREPARING FECAL SAMPLE, FECAL SAMPLE PREPARATION SOLUTION, AND FECES COLLECTION KIT**

(30) Priority: 26.08.2008 JP 2008217019
(71) Applicant: OLYMPUS CORPORATION, Shibuya-ku Tokyo 151-0072 (JP)
(72) Inventor: TANIGAMI Yasuo, Tokyo 151-0072 (JP); NAGAOKA Tomonori, Tokyo 151-0072 (JP)
(74) Representative: Schicker, Silvia
(86) International application number: PCT/JP2009/064789
(87) International publication number: WO 2010/024251

(57) **Abstract**

The present invention relates to the providing of a method for preparing a stool sample that enables nucleic acids in a stool to be stably preserved without requiring a complex procedure, a stool sample preparation solution and stool collection kit used in that method, and a method for recovering and analyzing nucleic acids in a stool using a stool sample prepared according to the preparation method of the present invention, and a stool sample having superior preservation of nucleic acids contained in the stool sample is prepared by mixing a collected stool with the stool sample preparation solution having for an active ingredient thereof a water-soluble organic solvent containing an organic acid.

## Description

### TECHNICAL FIELD

The present invention relates to a stool sample preparation method for preparing a stool sample having superior preservation of nucleic acids contained in a stool sample, a solution for preparation a stool sample and stool collection kit, a stool sample prepared according to that preparation method, a method for recovering nucleic acids from that stool sample, and a nucleic acid analysis method that uses nucleic acids recovered according to that nucleic acid recovery method.
The present application claims priority on the basis of Japanese Patent Application No. 2008-217019, filed in Japan on August 26, 2008, the contents of which are incorporated herein by reference.

### BACKGROUND ART

The number of colorectal cancer patients is currently continuing to increase rapidly each year in not only the U.S. and Europe, but in Japan as well, and is becoming one of the leading causes of cancer-related deaths. This is thought to be due to the growing proliferation of a Western style diet consisting primarily of red meat among the Japanese people. More specifically, roughly 60,000 persons are afflicted with colorectal cancer each year, and in terms of the number of deaths by organ, colorectal cancer is ranked third after gastric cancer and lung cancer, and is predicted to continue to increase in the future. On the other hand, differing from other forms of cancer, colorectal cancer has a nearly 100% cure rate if treated soon after onset. Thus, it is extremely significant to include colorectal cancer in early cancer screening examinations, and research and development of testing methods for early discovery of colorectal cancer is proceeding at a rapid pace.

Methods such as barium enema examinations and colonoscopies are performed as testing methods for early discovery of colorectal cancer. Barium enema examinations consist of injecting barium into the large intestine and allowing it to adhere to the mucosal membranes of the large intestine, irradiating the intestine with X-rays to capture images of any surface irregularities, and then observing the surface. On the other hand, colonoscopy consists of observing the inside of the large intestine directly with an endoscope. Colonoscopy in particular enables high levels of sensitivity and specificity, while also offering the advantage of allowing the excision of polyps and early forms of cancer.
However, in addition to be associated with high costs, these examinations place a considerable burden on the patient while also having the problem of being accompanied by complication risks. For example, barium enemas have risks associated with X-ray exposure and intestinal obstruction. In addition, colonoscopy is an invasive procedure since the endoscope is inserted directly into the large intestine. Moreover, the endoscopic procedure requires an experienced technician and the number of facilities where this examination can be performed is limited. Consequently, these examinations are not suitable for colorectal cancer examinations targeted at asymptomatic, healthy individuals as part of routine health examinations and the like.

In recent years, fecal occult blood tests have been widely performed as a non-invasive and inexpensive method for primary screening for colorectal cancer. The fecal occult blood test is a test for the presence of hemoglobin originating in erythrocytes contained in fecal matter, and is used as a method for indirectly predicting the presence of colorectal cancer. Factors behind the widespread use of the fecal occult blood test include stool samples being able to be collected and stored at room temperature eliminating the need for refrigerators, freezers and other special storage conditions, samples being able to be collected easily at home, and the test procedure being extremely simple. However, since the fecal occult blood test has low sensitivity of only about 25%, it has the problem of a high percentage of colorectal cancer being overlooked. Moreover, it also has a low positive predictive value, with the percentage of actual colorectal cancer patients among subjects judged to be positive in the fecal occult blood test being only about 10%, thus resulting in a large number of false positives. Consequently, there is a strong need for the development of a new examination method offering higher reliability.

Attention is currently focusing on new examination methods that are suitable for routine health examinations by being non-invasive, simple and highly reliable for use in testing for the presence of cancer cells and cancer cell-derived genes in stool samples. Since these examination methods investigate the presence of cancer cells or cancer cell-derived genes directly, they are considered to be more reliable than the fecal occult blood test, which tests for the presence of blood from the digestive tract that occurs indirectly accompanying the onset of colorectal cancer.

In order to accurately detect cancer cells and the like in stool samples, it is important to efficiently recover cancer cell-derived nucleic acids from those stool samples. In particular, since cancer cell-derived nucleic acids are only present in trace amounts in stool samples, and stool samples also contain large amounts of digestive remnants and bacteria, nucleic acids are decomposed extremely easily. Consequently, in order to efficiently recover nucleic acids, and particularly nucleic acids derived from mammalian cells such as human cells, from stool samples, it is important to prevent decomposition of nucleic acids within the stool and prepare the stool sample so that it can be stored stably until the time of the testing procedure. An example of such a stool sample processing method consists of separating cancer cells that have dissociated from the large intestine or other constituent of the digestive tract from a collected stool sample. Separation of cancer cells from stool makes it possible to inhibit the effects of bacterial proteases, DNase, RNase and other degrading enzymes. Examples of methods that have been disclosed for separating cancer cells from stool include: (1) a method for separating cells from stool, comprising: (a) a step for cooling the stool to a temperature below its gel freezing point, and (b) a step for collecting cells from the stool while maintaining at a temperature below the gel freezing point so that the stool substantially remains completely intact (see, for example, Patent Document 1). Another example of such a method consists of: (2) dispersing the stool in a transport medium containing a protease inhibitor, mucous dissolver and bactericide at a normal ambient temperature, followed by isolating the colorectal dissociated cells (see, for example, Patent Document 2).

On the other hand, numerous fixation methods, such as formalin fixation or alcohol fixation, have conventionally been employed to maintain the morphology of collected cells until the time of observation in cases of histological and cytological observation of cell morphology. A method that has been disclosed as an example of a method that applies these fixation methods consists of (3) a cell solution preservative comprising an alcohol that is miscible with an amount of water sufficient for colonizing mammalian cells, an amount of anti-aggregation agent sufficient for preventing aggregation of mammalian cells in the solution, and a buffer for maintaining the pH of the solution within the range of 4 to 7 during the time the cells are stored, which is used as a storage solution for enabling mammalian cell samples to be stored for long periods of time or enable cells to be observed following storage (see, for example, Patent Document 3).

In addition, disclosed examples of storage solutions that enable histological and cytological examinations of cells as well as molecular analyses of proteins or nucleic acids and the like present in cells after storage include (4) a universal collection medium containing a buffer component, at least one alcohol component, a fixative component and a chemical agent that inhibits decomposition of at least one member selected from the group consisting of RNA, DNA and protein (see, for example, Patent Document 4), and a non-aqueous solution containing 5 to 20% polyethylene glycol and 80 to 95% methanol (see, for example, Patent Document 5). In addition, (6) a composition has been disclosed for stabilizing cell structure and nucleic acids that comprises (a) a first substance capable of precipitating or denaturing protein containing at least one member of alcohol or ketone, and (b) a second promoting substance for promoting injection of the first substance into at least one cell (see, for example, Patent Document 6), and (7) a fixative composition has been disclosed for storing tissue and biological samples that comprises one or more alcohols, polyethylene glycol having a molecular weight of 200 to 600, one or more weak organic acids mixed at a concentration of 0.01 to 0.10 mole per liter of the fixative composition, and water, wherein the fixative composition is substantially free of a crosslinking binder such as formaldehyde (see, for example, Patent Document 7).

### [Prior Art Documents]

### [Patent Documents]

[Patent Document 1] Japanese Translation of PCT Application No. H11-511982
[Patent Document 2] Japanese Translation of PCT Application No. 2004-519202
[Patent Document 3] Japanese Unexamined Patent Application, First Publication No. 2003-153688
[Patent Document 4] Japanese Translation of PCT Application No. 2004-500897
[Patent Document 5] Japanese Translation of PCT Application No. 2005-532824
[Patent Document 6] Japanese Unexamined Patent Application, First Publication No. 2001-128662
[Patent Document 7] Japanese Translation of PCT Application No. 2008-502913

### SUMMARY OF THE INVENTION

### [Problems to be Solved by the Invention]

In the method described in (1) above, cells are separated while cooling the stool sample. If this separation procedure is carried out without cooling, correct detection results are no longer able to be obtained due to deterioration of the stool sample. Thus, in order to effectively prevent deterioration of the stool sample, it is important to cool the stool sample immediately after collecting. However, in the case of collecting a stool sample at home as is done in the case of routine health examinations and the like, it is extremely difficult and unrealistic to promptly cool the stool sample after collection.
In addition, although it is possible to freeze the stool sample to prevent deterioration, a frozen stool sample must be thawed prior to testing, thereby making the procedure excessively complex.

In the method described in (2) above, although this method does not require a cooling procedure and allows preparation and storage of stool samples at room temperature as a result of adding a bactericide and the like, it has the problem of the procedure for separating cells that have dissociated from the large intestine from stool being complex. In addition, there is the risk of a decrease in the detection accuracy of colorectal cancer since large intestine dissociated cells and nucleic acids and the like derived from large intestine dissociated cells end up being decomposed by nucleases and proteases derived from bacteria destroyed by the bactericide and the like. In addition, since the cells are stored in a viable state, there is also the problem of gene expression and other molecular profiling of the large intestine dissociated cells changing as a result of being affected by antibiotics and other components in the medium as well as changes over time.

In addition, cells can be stably stored at room temperature by using the storage solutions described in (3) to (5) above. However, these storage solutions are used for nearly isolated cells, and encounter difficulties when used directly for biological samples such as stool samples containing various substances. In addition, the composition described in (6) above is used to stably store nucleic acids primarily derived from bacteria present in vaginal swab samples. There is no description whatsoever regarding whether the composition described in (6) above is a composition that enables the stable storage of nucleic acids derived from mammalian cells, which have a considerably different structure from that of bacteria and which are present in much smaller amounts than bacteria. In addition, even if the composition described in (6) above is used for stool containing digestive remnants that differs from vaginal swab samples, there is no description whatsoever as to whether nucleic acids can be stored stably. Similarly, the composition described in (7) above is a composition used as a fixative for specimens and other samples having extremely low levels of contaminants, and there is no description whatsoever regarding whether this composition enables stable storage of nucleic acids even in cases in which it is used for stool samples containing large amounts of digestive remnants and the like.

An object of the present invention is to provide a method for preparing a stool sample enabling nucleic acids present in stool to be stored stably without requiring a complex procedure, a solution for preparing a stool sample and a stool collection kit used in that preparation method, and a method for recovering and analyzing nucleic acids present in stool by using a stool sample prepared according to that preparation method.

### [Means for Solving the Problems]

The present invention was completed by achieving the following in order to solve the aforementioned problems:
I. a stool sample enabling stable storage of nucleic acids contained in stool was able to be prepared by mixing a collected stool in a solution for preparing a stool sample having as an active ingredient thereof a water-soluble organic solvent containing an organic acid; and
II. nucleic acids present in trace amounts derived from biological matter (herein after referred as creature) other than indigenous intestinal flora (herein after specified as intestinal bacteria) were also able to be recovered extremely efficiently by simultaneously recovering nucleic acids derived from creature other than indigenous intestinal bacteria such as mammalian cells to be detected and nucleic acids derived from indigenous intestinal bacteria contained in large amounts in stool from a stool sample prepared according to that preparation method.

Namely, the present invention is as described below.
(1) A stool sample preparation method, comprising: mixing a collected stool with a solution for preparing a stool sample.

(2) In addition, the present invention is a solution for preparing a stool sample that is used to mix a collected stool and has a water-soluble organic solvent containing an organic acid, the water-soluble organic solvent being as an active ingredient of the solution.

(3) The solution for preparing a stool sample described in (2) above may also have a buffering action.

(4) The water-soluble organic solvent contained in the solution for preparing a stool sample described in (2) or (3) above may be one or more members selected from the group consisting of a water-soluble alcohol, ketone and aldehyde.

(5) The pH of the solution for preparing a stool sample described in any of (2) to (4) above may be 2 to 6.5.

(6) The pH of the solution for preparing a stool sample described in (5) above may be 3 to 6.

(7) The organic acid contained in the solution for preparing a stool sample described in any of (2) to (6) above may be one or more members selected from the group consisting of a linear aliphatic acid, dicarboxylic acid, amino acid, hydroxy acid and aromatic or heterocyclic polycarboxylic acid.

(8) The organic acid contained in the solution for preparing a stool sample described in (7) above may be one or more members selected from the group consisting of acetic acid, lactic acid, citric acid and adipic acid.

(9) The pH of the solution for preparing a stool sample described in any of (6) to (8) above may be 4.5 to 5.5.

(10) The organic acid concentration in the solution for preparing a stool sample described in any of (2) to (9) above may be 0.01 to 0.1 M.

(11) The water-soluble organic solvent contained in the solution for preparing a stool sample described in any of (4) to (10) above may be one or more of a water-soluble alcohol and ketone, and the concentration of the water-soluble organic solvent may be 30% or more.

(12) The water-soluble organic solvent contained in the solution for preparing a stool sample described in (11) above may contain one or more members selected from the group consisting of ethanol, propanol and methanol as water-soluble alcohol.

(13) The water-soluble organic solvent contained in the solution for preparing a stool sample described in (11) above may be ethanol.

(14) The water-soluble organic solvent contained in the solution for preparing a stool sample described in (11) or (12) above may contain one or more of acetone and methyl ethyl ketone as ketone.

(15) The water-soluble organic solvent contained in the solution for preparing a stool sample described in any of (4) to (10) above is an aldehyde, and the concentration of this water-soluble organic solvent is within a range of 0.01 to 30%.

(16) In the solution for preparing a stool sample described in any of (2) to (15) above, the mixing ratio of the stool and the solution for preparing a stool sample is such that the ratio of the volume of the solution for preparing a stool sample is 1 or more based on a value of 1 for the volume of the stool.

(17) The solution for preparing a stool sample described in any of (2) to (16) above may also contain a detergent.

(18) The solution for preparing a stool sample described in any of (2) to (17) above may also contain a colorant.

(19) The concentration of the water-soluble organic solvent in the solution for preparing a stool sample described in any of (2) to (18) above may be 30% or more.

(20) In addition, the present invention is a stool collection kit, comprising: a solution for preparing a stool sample having a water-soluble organic solvent that contains an organic acid, the water-soluble organic solvent being as an active ingredient of the solution, and a stool collection container containing the solution for preparing a stool sample.

(21) The concentration of the water-soluble organic solvent containing organic acid in the solution for preparing a stool sample included in stool collection kit described in (20) above may be 30% or more.

(22) In addition, the present invention is a stool sample prepared using the solution for preparing a stool sample described in any of (2) to (19) above.

(23) In addition, the present invention is a method for recovering nucleic acids from a stool sample, wherein nucleic acids derived from indigenous intestinal bacteria and nucleic acids derived from creature other than indigenous intestinal bacteria are recovered simultaneously from the stool sample described in (22) above.

(24) In the nucleic acid recovery method described in (23) above, the nucleic acids derived from the creature other than indigenous intestinal bacteria may be nucleic acids derived from mammalian cells.

(25) A step for recovering nucleic acids in the nucleic acid recovery method described in (23) or (24) above may include (a) a step for denaturing protein in the stool sample and eluting nucleic acids from the indigenous intestinal bacteria and the creature other than indigenous intestinal bacteria in the stool sample, and (b) a step for recovering the nucleic acids eluted in the step (a).

(26) In the nucleic acid recovery method described in (25) above, (c) a step for removing the protein denatured by the step (a) may be included after the step (a) and before the step (b).

(27) Denaturation of protein in the step (a) in the nucleic acid recovery method described in (25) or (26) above may be carried out using one or more members selected from the group consisting of a chaotropic agent, an organic solvent and a detergent.

(28) The organic solvent used in the nucleic acid recovery method described in (27) above may be phenol.

(29) Removal of denatured protein in the step (c) in the nucleic acid recovery method described in any of (26) to (28) above may be carried out using chloroform.

(30) Recovery of nucleic acids in the step (b) in the nucleic acid recovery method described in any of (25) to (29) above may include (b1) a step for adsorbing the nucleic acids eluted in the step (a) to an inorganic support, and (b2) a step for eluting the nucleic acids adsorbed in the step (b1) from the inorganic support.

(31) The nucleic acid recovery method described in any of (25) to (30) above may also include (d) a step for recovering a solid component from the stool sample before the step (a).

(32) In addition, the present invention is a method for analyzing nucleic acids derived from mammalian cells using nucleic acids recovered using the nucleic acid recovery method described in any of (23) to (31) above.

(33) The mammalian cells used in the nucleic acid analysis method described in (32) above may be gastrointestinal cells.

(34) The mammalian cells used in the nucleic acid analysis method described in (32) above may be large intestine dissociated cells.

(35) Nucleic acids derived from the mammalian cells used in the nucleic acid analysis method described in any of (32) to (34) may be markers indicating a neoplastic transformation.

(36) Nucleic acids derived from the mammalian cells used in the nucleic acid analysis method described in any of (32) to (34) may be markers indicating an inflammatory gastrointestinal disease.

(37) The nucleic acid analysis method described in any of (32) to (36) above may be one or more of RNA analysis and DNA analysis.

(38) In the nucleic acid analysis method described in (37) above, the RNA analysis may consist of one or more of an analysis of an insertion, deletion, substitution, duplication or inversion of a base in the RNA or a splicing variant, an mRNA expression analysis and a functional RNA analysis.

(39) In the nucleic acid analysis method described in (37) above, the DNA analysis may be one or more of a mutation analysis and an analysis of epigenetic changes.

(40) In the nucleic acid analysis method described in (39) above, the mutation analysis may be an analysis of one or more mutations of an insertion, deletion, substitution, duplication or inversion of a base.

(41) In the nucleic acid analysis method described in (39) above, the analysis of epigenetic changes may be one or more of a DNA methylation analysis and DNA demethylation analysis.

(42) In the nucleic acid analysis method described in (39) above, the mutation analysis may be a mutation analysis of K-ras gene.

### [Effects of the Invention]

The solution for preparing a stool sample of the present invention has superior preservation of nucleic acids contained in a stool sample, and has for an active ingredient thereof a water-soluble organic solvent containing organic acid. By mixing a stool into the water-soluble organic solvent containing organic acid, loss of nucleic acids contained in the stool due to decomposition and the like can be held to a minimum, and nucleic acids can be stored extremely stably in the water-soluble organic solvent. This high nucleic acid preservation effect inhibits changes in nucleic acids over time by considerably lowering the cellular activity of the creature having nucleic acids such as indigenous intestinal bacteria, mammalian cells or viruses due to dehydrating action possessed by the water-soluble organic solvent. In addition, the high nucleic acid preservation effect of the solution for preparing a stool sample of the present invention is presumed to be the result of nucleic acid decomposition being inhibited by a considerable decrease in the activities of various decomposing enzymes such as protease, DNase or RNase present in the stool due to the protein denaturing action possessed by the water-soluble organic solvent component. Moreover, since the solution for preparing a stool sample of the present invention has for an active ingredient thereof a water-soluble organic solvent containing organic acid, the solution for preparing a stool sample can be maintained in an acidic state. Consequently, the protein denaturing action of the water-soluble organic solvent component can be further enhanced. Moreover, extremely superior nucleic acid preservation effects are presumed to be obtained since hydrolysis and the like of nucleic acids can also be effectively inhibited.

In addition, a stool sample in which nucleic acids present in stool can be stored stably can be prepared according to the stool sample preparation method of the present invention. Namely, according to the stool sample preparation method of the present invention, nucleic acids derived from the creature other than indigenous intestinal bacteria such as mammalian cells contained in comparative small amounts in stool samples can be maintained in a stable state that enables them to be stored for a long period of time at room temperature. In this manner, use of the stool sample preparation method of the present invention enables collection of stool to preparation, storage and transport of a stool sample to be carried out easily at room temperature while stably storing nucleic acids present in the stool sample, thereby making this extremely preferable for preparation of a stool sample for use in routine health examinations and other screening examinations. Moreover, even in the case of preparing a stool sample for analysis of nucleic acids derived from the creature other than indigenous intestinal bacteria such as mammalian cells, since there is no need for a complex procedure involving separation of the creature or cells thereof and the like, on which detection of mammalian cells and the like is to be carried out, from the stool sample, even in cases of processing a large number of specimens, both labor and costs can be effectively reduced. In particular, a stool sample can be prepared even more easily by using the stool collection kit of the present invention.

### BRIEF DESCRIPTION OF THE DRAWINGS

FIG. 1 is a drawing showing an embodiment of a stool collection container A that can be used in the stool collection kit of the present invention.
FIG. 2 is a drawing showing an embodiment of a stool collection container B that can be used in the stool collection kit of the present invention, and an example of its method of use.
FIG. 3 is a graph showing the results of a relative comparison of expression levels of GAPDH gene in RNA derived from each of the stool samples in Example 1.
FIG. 4 is a graph showing the results of a relative comparison of expression levels of GAPDH gene in RNA derived from stool samples 2-1 to 2-3 and 5-1 in Example 2.
FIG. 5 is a graph showing the results of a relative comparison of expression levels of GAPDH gene in RNA derived from stool samples 3-1 to 3-3 and 5-1 in Example 2.
FIG. 6 is a graph showing the results of a relative comparison of expression levels of GAPDH gene in RNA derived from stool samples 4-1 to 4-3 and 5-1 in Example 2.
FIG. 7 is a graph showing amounts of RNA recovered from each of the stool samples in Reference Example 1.
FIG. 8 is a graph showing amounts of RNA recovered from stool samples prepared using ethanol solutions of various concentrations in Reference Example 3.

### BEST MODE FOR CARRYING OUT THE INVENTION

The solution for preparing a stool sample used in the stool sample preparation method of the present invention (to be abbreviated as preparation solution S), or in other words, the preparation solution S of the present invention, has for an active ingredient thereof a water-soluble organic solvent containing organic acid. Since biological samples such as stool normally have high moisture contents, solvent having high water solubility or a water-soluble organic solvent capable of mixing at an arbitrary ratio with water is used as an active ingredient. As a result, the preparation solution S of the present invention is able to rapidly mix with a stool sample, thereby allowing the obtaining of higher nucleic acid preservation effects.

The water-soluble organic solvent in the present invention includes alcohols, ketones and aldehydes. In addition, the water-soluble organic solvent has a linear structure and is a liquid at room temperature such as at a temperature of 15 to 40°C. The use of a water-soluble organic solvent having a linear structure as an active ingredient enables mixing with stool to be carried out more rapidly than the use of an organic solvent having a cyclic structure such as benzene as an active ingredient.
Since organic solvents having a cyclic structure typically easily separate from water, they are difficult to mix with stool and make it difficult to obtain high nucleic acid preservation effects. This is because, even in the case of a solvent that dissolves to a certain degree in water, vigorous mixing or heating is required to uniformly disperse the stool. Furthermore, it is possible to first produce a mixed solvent of organic solvent and water and then mix the stool with the mixed solvent in order to facilitate mixing of stool with an organic solvent having a cyclic structure. However, it is necessary to vigorously mix or heat the organic solvent having a cyclic structure and the water in order to produce the mixed solvent, thereby making this undesirable.

The preparation solution S of the present invention is preferably a water-soluble organic solvent in which the solubility with respect to water is 12% by weight or more, more preferably that in which the solubility with respect to water is 20% by weight or more, even more preferably that in which the solubility with respect to water is 90% by weight or more, and particularly preferably a water-soluble organic solvent that can be mixed with water at an arbitrary ratio. Examples of water-soluble organic solvents that can be mixed with water at an arbitrary ratio include methanol, ethanol, n-propanol, 2-propanol, acetone and formaldehyde.

There are no particular limitations on the water-soluble organic solvent contained in the preparation solution S of the present invention provided it is a solvent that demonstrates a high nucleic acid preservation effect. Examples include alcohols such as water-soluble alcohols in the manner of methanol, ethanol, propanol, butanol or mercaptoethanol, ketones such as acetone or methyl ethyl ketone (having solubility with respect to water of 90% by weight or more), and aldehydes such as acetoaldehyde (acetylaldehyde), formaldehyde (formalin), glutaraldehyde, para-formaldehyde or glyoxal. The propanol may be n-propanol or 2-propanol. In addition, the butanol may be 1-butanol (having solubility with respect to water of 20% by weight) or 2-butanol (having solubility with respect to water of 12.5% by weight). Water-soluble alcohols, acetone, methyl ethyl ketone or formaldehyde is preferably used for the water-soluble organic solvent used in the present invention since these have sufficiently high solubility with respect to water. Water-soluble alcohols are more preferable, and ethanol, propanol or methanol is even more preferable, from the viewpoints of availability, handling ease, safety and the like. In particular, ethanol is particularly useful in routine health examinations and other screening examinations since it has the highest degree of safety and can be handled easily even in the home.

There are no particular limitations on the concentration of the water-soluble organic solvent in the preparation solution S of the present invention provided it is a concentration that demonstrates high nucleic acid preservation effects, and can be suitably determined in consideration of the type (member) of water-soluble organic solvent and the like. For example, in the case of using a water-soluble alcohol or ketone as an active ingredient, the concentration of the water-soluble organic solvent of the preparation solution S of the present invention is preferably 30% or more. By making the concentration of the water-soluble organic solvent adequately high, the water-soluble organic solvent is able to rapidly penetrate into mammalian cells or indigenous intestinal bacteria in the stool in the case of mixing the stool with the preparation solution S, thereby enabling high nucleic acid preservation effects to be demonstrated rapidly.
Furthermore, in the present invention and description of the present application, the term "%" refers to "% by volume (vol%)" unless specifically indicated otherwise.

In the case of using a water-soluble alcohol for the active ingredient in particular, the concentration of the water-soluble organic solvent of the preparation solution S of the present invention is preferably 30% or more, more preferably 50% or more, even more preferably within the range of 50 to 80% and particularly preferably within the range of 60 to 70%. If the concentration of the water-soluble organic solvent is high, high nucleic acid preservation effects can be adequately obtained while requiring only a small amount of the preparation solution S even for stool having high water content.

In addition, in the case of using acetone or methyl ethyl ketone for the active ingredient, the concentration of the water-soluble organic solvent of the preparation solution S of the present invention is preferably 30% or more, more preferably 60% or more and even more preferably 80% or more. In addition, in the case of using acetaldehyde, formaldehyde, glutaraldehyde, para-formaldehyde or glyoxal for the active ingredient, the concentration of the water-soluble organic solvent of the preparation solution S of the present invention is preferably within the range of 0.01 to 30%, more preferably within the range of 0.03 to 10% and even more preferably within the range of 3 to 5%. Aldehydes are able to demonstrate high nucleic acid preservation effects at lower concentrations than alcohols or ketones.

In addition, the water-soluble organic solvent used in the present invention may contain only one member of water-soluble organic solvent or may be a mixed solution of two or more members of water-soluble organic solvents. For example, the water-soluble organic solvent may be a mixed solution of two or more members of alcohols, or a mixed solution of an alcohol and another member of water-soluble organic solvent. A mixed solution of alcohol and acetone is preferable since nucleic acid storage efficiency is further improved.

Examples of the organic acid used in the present invention include linear aliphatic acids, dicarboxylic acids, amino acids, hydroxy acids and aromatic or heterocyclic polycarboxylic acids. Specific examples include linear aliphatic acids such as formic acid, acetic acid, propionic acid, butyric acid or valeric acid; dicarboxylic acids such as oxalic acid, malonic acid, succinic acid, glutaric acid, adipic acid, fumaric acid, maleic acid, phthalic acid, isophthalic acid or terephthalic acid; amino acids such as glycine, alanine, methionine, serine, valine, leucine, isoleucine, threonine, cysteine, cystine, phenylalanine, glutamic acid or aspartic acid; hydroxy acids such as glycolic acid, lactic acid, hydroxyacrylic acid, α-oxybutyric acid, glyceric acid, tartronic acid, malic acid, tartaric acid, citric acid, salicylic acid, gallic acid, mandelic acid, tropic acid, ascorbic acid or gluconic acid; and aromatic or heterocyclic polycarboxylic acids such as cinnamic acid, benzoic acid, phenylacetic acid, nicotinic acid, kainic acid, sorbic acid, pyrrolidone carboxylic acid or trimellitic acid.
Linear aliphatic acids, dicarboxylic acids or hydroxy acids and the like are particularly preferable for use as the organic acid added to the preparation solution S of the present invention, while acetic acid, adipic acid, citric acid or lactic acid is more preferable. The use of adipic acid or citric acid allows the obtaining of particularly superior nucleic acid preservation effects. In addition, acetic acid is particularly preferable since it allows the obtaining of adequate nucleic acid preservation effects while also being widely used and economical.

Furthermore, the preparation solution S of the present invention may contain only one member of organic acid or may contain two or more members of organic acids. In addition, there are no particular limitations on the amount of the organic acid added to the preparation solution S of the present invention provided the preparation solution S can be maintained in an acidic state, and can be suitably determined in consideration of the member of organic acid added, the member and concentration of the water-soluble organic solvent in the preparation solution S and the like.

The pH of the preparation solution S of the present invention is preferably acidic. This is to more effectively inhibit hydrolysis of nucleic acids. The pH of the preparation solution S of the present invention is preferably within the range of 2 to 6.5, more preferably within the range of 3 to 6, and even more preferably within the range of 4.5 to 5.5.

In the case a certain amount of acid or base has been added, and particularly in the case stool has been added, the preparation solution S of the present invention preferably has buffering action so that the pH thereof is maintained within the aforementioned pH ranges with little fluctuation in pH. Although that in which an organic acid and water-soluble organic solvent have been added to a suitable buffer solution may be used for the preparation solution S having buffering action, in the present invention, the preparation solution S particularly preferably contains an organic acid and a conjugate base of that organic acid and demonstrates buffering action attributable to the organic acid and the conjugate base thereof. For example, the preparation solution S may be adjusted to a desired pH by adding an organic acid and an alkaline metal salt or alkaline earth metal salt of that organic acid. In addition, pH may also be adjusted by using a hydroxide of an alkaline metal or alkaline earth metal after adding the organic acid.

In addition, the preparation solution S of the present invention may be a solution that contains both organic acid and mineral acid and has suitable buffering action. For example, the preparation solution S may be a solution obtained by mixing a water-soluble organic solvent with a buffer system having buffering action in the acidic range such as a glycine/HCl buffer system, sodium cacodylate/HCl buffer system or potassium hydrogen phthalate/HCl buffer system.

Consequently, in the present invention, the organic acid concentration of the preparation solution S is preferably that which enables the adequate demonstration of buffering action. For example, adequate buffering action can be provided by adding organic acid so that the final concentration in the preparation solution S is within the range of 0.01 to 0.1 M.

Furthermore, in the present invention, the pH of the preparation solution S is the value obtained by measuring with a pH meter using the glass electrode method for the measuring principle thereof (such as that manufactured by DKK-Toa Corp.) after having calibrated with a phthalate standard solution and neutral phosphate standard solution.

Although there are no particular limitations on the volume of the preparation solution S added to a collected stool, if the volume of stool is assigned a value of 1, then the mixing ratio between the volume of stool and the volume of the preparation solution S is preferably such that the volume of the removal solution S is 1 or more based on the value of 1 for the volume of stool. In the case of placing the stool in a stool collection container containing the preparation solution S, the entire circumference of the stool can be immersed in the preparation solution S if the volume of the preparation solution S is equal to or greater than the volume of the stool, thereby enabling the effects of the present invention to be obtained. For example, in the case the volume of the stool is equal to the volume of the preparation solution S, the stool collection container containing the preparation solution S can be made to be lightweight and compact. On the other hand, by mixing a volume of the preparation solution S that is equal to or greater than 5 times the volume of the stool, the stool can be rapidly and effectively dispersed in the preparation solution S, and effects on the stool attributable to decreases in the concentration of water-soluble alcohol caused by water contained in the stool can be inhibited. Since a proper balance can be obtained between both the effects of reducing the weight of the stool collection container containing the preparation solution S and improving dispersibility of the stool, the mixing ratio of the stool to the preparation solution S is preferably within the range of 1:1 to 1:20, more preferably within the range of 1:3 to 1:10 and even more preferably about 1:5.

Furthermore, although there are no particular limitations on the stool used in the stool sample preparation method of the present invention provided it is of animal origin, it is preferably of mammalian origin and more preferably of human origin. For example, although it is preferably a human stool collected for the purpose of a routine health examination or diagnosis and the like, it may also be a stool from livestock or wild animal. In addition, although the stool may be that which has been stored for a fixed period of time after collection, it is preferably that immediately after collection. Moreover, although the collected stool is preferably obtained immediately after voiding, that for which time has elapsed after voiding may also be used.

Although there are no particular limitations on the amount of the stool used in the stool sample preparation method of the present invention, it is preferably within the range of 10 mg to 1 g. If the amount of the stool is excessively large, the collection procedure becomes difficult and the size of the collection container also increases, thereby resulting in the risk of a decrease in handling ease and the like. Conversely, in the case the amount of the stool is excessively small, since the number of large intestine dissociated cells and other mammalian cells contained in the stool becomes excessively low, the necessary amount of nucleic acid cannot be recovered, thereby resulting in the risk of a decrease in the target accuracy of nucleic acid analysis. In addition, since stool is heterogeneous, or in other words, numerous members and forms of components are non-uniformly present therein, the stool sample is preferably collected from a wide range of the stool at the time of stool collection to avoid the effect of localization of mammalian cells.

The removal solution S is obtained by adding a suitable amount of organic acid after suitably diluting the water-soluble organic solvent and adjusting to a desired concentration. Although there are no particular limitations on the solvent used for dilution, water or a buffer such as phosphate buffer is preferable. For example, the preparation solution S having preferable buffering action can be obtained by suitably diluting the water-soluble organic solvent with water followed by adding an adequate amount of organic acid, and then adjusting the pH to a desired pH using a hydroxide solution of an alkaline metal or alkaline earth metal such as sodium hydroxide.

In addition, the preparation solution S of the present invention may also contain arbitrary components in addition to the organic acid and water-soluble organic solvent provided they do not impair the high nucleic acid preservation effects of the water-soluble organic solvent component. For example, the preparation solution S may contain a chaotropic salt or a detergent. The containing of a chaotropic salt or detergent makes it possible to more effectively inhibit cellular activity and enzyme activity of various decomposing enzymes present in the stool. Examples of chaotropic salts that can be added to the preparation solution S include guanidine hydrochloride, guanidine isothiocyanate, sodium iodide, sodium perchlorate and sodium trichloroacetate. A nonionic detergent is preferable for the detergent able to be added to the preparation solution S.
Examples of these nonionic detergents include Tween 80, CHAPS 3-[3-cholamidopropyl-dimethylammonio]-1-propane sulfonate), Triton X-100 and Tween 20. There are no particular limitations on the member and concentration of the chaotropic salt or detergent provided they are at a concentration that allows the obtaining of high nucleic acid preservation effects, and can be suitably determined in consideration of the amount of stool and subsequent processing steps, analysis methods and the like.

In addition, a suitable colorant may be added to the preparation solution S. Coloring the preparation solution S allows the obtaining of effects such as prevention of accidental swallowing and reducing stool color. The colorant is preferably a colorant used as a food additive, and is preferably blue or green and the like. Examples of colorants include Fast Green FCF (Green No. 3), Brilliant Blue FCF (Blue No. 1) and Indigo Carmine (Blue No. 2). In addition, a plurality of colorants may be used as a mixture or a single colorant may be added alone.

The stool and the preparation solution S are preferably mixed rapidly. This is because, by rapidly dispersing the stool in the preparation solution S, the water-soluble organic solvent is able to rapidly penetrate into cells in the stool, thereby high nucleic acid preservation effects to be obtained quickly.
Furthermore, there are no particular limitations on the method used to mix the stool and preparation solution S provided it is a method that involves mixing by physical means. For example, after having placed a collected stool in a sealable container preliminarily containing the preparation solution S and sealing the container, the stool and the preparation solution S may be mixed by vertically inverting the container or by placing the container on an agitator such as a vortex. In addition, the stool and the preparation solution S may also be mixed in the presence of mixing particles.
A method that uses an agitator or a method that uses mixing particles is preferable for this mixing method since mixing can be carried out rapidly. In particular, the use of a stool collection container preliminarily containing mixing particles enables mixing to be carried out rapidly even in an environment such as the home where there are no special equipment.

There are no particular limitations on the mixing particles provided they are of a composition that does not impair the high nucleic acid preservation effects of the water-soluble organic solvent component, and have a particle size and specific gravity that allows the stool to be rapidly dispersed in the preparation solution S by colliding with the stool. Moreover, the mixing particles may be particles composed of one member of material or may be particles composed of two or more members of materials. Examples of such mixing particles include particles composed of glass, ceramics, plastic, latex or metal. In addition, the mixing particles may be magnetic particles or non-magnetic particles.

Stool is preferably prepared using the preparation solution S of the present invention in the case of analyzing as target nucleic acids those nucleic acids derived from the creature other than indigenous intestinal bacteria, namely nucleic acids which are contained in much smaller amounts than nucleic acids derived from indigenous intestinal bacteria contained in large amounts in stool samples. Nucleic acids in stool are gradually lost with the passage of time after voiding due to decomposition and the like. Consequently, in the case of a target nucleic acid which is present in only a small amount in the stool, an amount of the target nucleic acid adequate for analysis cannot be recovered if analysis is carried out using a stool sample in which decomposition of nucleic acids has progressed. As a result, there is the risk of a stool sample being judged negative (absence of the target nucleic acid in the stool) even if the target nucleic acid is present in the stool immediately after voiding. Since nucleic acids present in stool can be stored stably by preparing the stool using the preparation solution S of the present invention, even nucleic acids present only in small amounts in the stool can be efficiently recovered. Accordingly, the reliability of nucleic acid analysis can be improved.

Examples of nucleic acids derived from the creature other than indigenous intestinal bacteria include nucleic acids derived from mammalian cells such as nucleic acids derived from cancer cells, and nucleic acids derived from causative organisms of infectious diseases in the early stage or late stage of those infectious diseases such as hepatitis virus. In addition, they may also be nucleic acids derived from parasites.

Furthermore, in the present invention, indigenous intestinal bacteria refer to bacterial cells present in comparatively large amounts in stool, and indicates indigenous bacteria that normally thrives in the intestines of animals such as humans. Examples of indigenous intestinal bacteria include obligatory anaerobic bacteria such as Bacteroides species, Eubacterium species, Bifidobacterium species or Clostridium species, and facultative anaerobic bacteria such as Escherichia species, Enterobacter species, Klebsiella species, Citrobacter species or Enterococcus species.

The high nucleic acid preservation effects of the water-soluble organic solvent component as described above are not particularly subjected to the effects of temperature conditions provided an adequate amount of the water-soluble organic solvent is present. Thus, the stool sample preparation method of the present invention enables nucleic acids to be stably stored in a stool sample even in the case of carrying out preparation at a temperature at which stool collection is normally carried out, namely even in the case of carrying out preparation at room temperature. In addition, the prepared stool sample is able to stably preserve nucleic acids in the stool sample even in the case of storing or transporting at room temperature. However, the storage temperature of the stool sample is preferably 50°C or lower. This is because there is the risk of the concentration of the water-soluble organic solvent in the stool sample decreasing to a concentration lower than an adequate concentration required to demonstrate high nucleic acid preservation effects due to volatilization and the like.

A stool sample prepared according to the stool sample preparation method of the present invention, or in other words, the stool sample of the present invention, is able to more stably preserve nucleic acids in stool, and particularly nucleic acids derived from mammalian cells and the like which are only present in comparatively small amounts in stool, due to the dehydrating action, protein denaturing action and nucleic acid decomposition inhibitory action of the water-soluble organic solvent containing organic acid. Consequently, in the case of preparing a stool sample according to the preparation method of the present invention, even if nucleic acid analyses are carried out using not only a stool sample immediately after preparation, but also using a stool sample following long-term storage or transport, highly reliable analysis results can be expected to be obtained. In particular, nucleic acids present in stool, and particularly nucleic acids derived from mammalian cells, can be stable preserved at room temperature for an extended period of time while minimizing changes over time in molecular profiling of mammalian cells such as exfoliated colonocytes contained in the stool. Consequently, by preparing a collected stool using the preparation method of the present invention, even in cases in which time is required from stool collection to nucleic acid analysis or in cases in which the location where the stool sample is collected is a considerable distance away from the location where nucleic acids are analyzed as is the case with routine health examinations or screening examinations and the like, the stool sample can be stored or transported while inhibiting decomposition of nucleic acids, and particularly decomposition of fragile RNA. In addition, special equipment for refrigerating or freezing and the setting of storage temperature conditions are not required, and stool samples can be stored or transported easily and at low cost.

The stool sample of the present invention can be applied to various nucleic acid analyses in the same manner as other biological samples containing nucleic acids. It is particularly preferably used in nucleic acid analyses for investigating the presence or absence of the onset of cancer or infectious diseases for which early detection is important. In addition, it is preferably used in nucleic acid analyses for investigating for the presence or absence of the onset of inflammatory diseases such as colitis, enteritis, gastritis or pancreatitis. It may also be used for testing for protruding lesions such as polyps as well as testing for diseases of the large intestine, small intestine, stomach, liver, gallbladder and bile duct, such as gastric ulcer.

For example, the presence or absence of the onset of colon cancer, pancreatic cancer or other cancers can be examined by detecting and analyzing nucleic acids derived from cancer cells, namely nucleic acids in which mutations and the like are occurring, in a stool sample. In addition, the onset of infection or the presence of parasites can be investigated by investigating whether or not nucleic acids derived from a pathogenic organism causing the infection, such as viral nucleic acids or parasite-derived nucleic acids, are detected in a stool sample. In particular, testing for infections can be carried out both non-invasively and easily by a stool sample to detect pathogenic organisms excreted into the stool, such as hepatitis A virus or hepatitis E virus. In addition, presence or absence of the onset of a bacterial infection can be investigated by detecting nucleic acids derived from pathogenic bacteria other than indigenous intestinal bacteria, such as bacteria causing food poisoning or pathogenic microorganisms such as enterohemorrhagic Escherichia coli O-157.

In particular, a marker indicating a neoplastic transformation or a marker indicating an inflammatory gastrointestinal disease is preferably detected by nucleic acid analysis. Examples of markers indicating neoplastic transformation include known cancer markers such as carcinoembryonic antigen (CEA) or sialyl Tn antigen (STN), and mutations such as those of APC gene, p53 gene or K-ras gene. In addition, detection of methylation of genes such as p16, hMLH1, MGMT, p14, APC, E-cadherin, ESR1 or SFRP2 is also useful as a diagnostic marker of colon diseases (see, for example, Lind, et al., "A CpG island hypermethylation profile of primary colorectal carcinomas and colon cancer cell lines", Molecular Cancer, 2004, Vol. 3, Chapter 28). In addition, DNA derived from Helicobacter pylori present in a stool sample has been previously reported to be used as a stomach cancer marker (see, for example, Nilsson, et al., Journal of Clinical Microbiology, 2004, Vol. 42, No. 8, pp. 3781-8). On the other hand, an example of a marker that indicates an inflammatory gastrointestinal disease is nucleic acid derived from Cox-2 gene.

Since nucleic acids can be recovered extremely efficiently from a stool sample prepared according to the preparation method of the present invention, this stool sample is an extremely preferable sample for analysis of not only nucleic acids derived from indigenous intestinal bacteria present in large amounts in stool, but also nucleic acids derived from mammalian cells present only in small amounts. In particular, since the sample is a stool sample, it is preferable for analyzing nucleic acids derived from digestive tract cells such as those of the large intestine, small intestine or stomach, and is particularly preferable for analyzing nucleic acids derived from large intestine dissociated cells.

A diverse range of various substances are present in stool samples, and numerous substances that act as inhibitory factors in nucleic acid analysis are also present therein. Consequently, by recovering nucleic acids from a stool sample and carrying out nucleic acid analysis using the recovered nucleic acids instead of carrying out nucleic acid analysis directly on the stool sample, the accuracy of the analysis can be further improved. There are no particular limitations on the method used to recover nucleic acids from a stool sample, and any method can be used provided it is a method that is normally used in the case of recovering nucleic acids from a sample. Nucleic acids derived mainly from the creature other than indigenous intestinal bacteria such as mammalian cells (to be referred to as mammalian cells) and nucleic acids derived from indigenous intestinal bacteria are contained in the stool sample of the present invention. In recovering nucleic acids from the stool sample, although nucleic acids derived from mammalian cells and nucleic acids derived from indigenous intestinal bacteria may be recovered separately, they are particularly preferably recovered simultaneously. Simultaneously recovering nucleic acids derived from mammalian cells and nucleic acids derived from indigenous intestinal bacteria allows nucleic acids derived from indigenous intestinal bacteria present in large amounts in stool to function as carriers. As a result, nucleic acids derived from mammalian cells present in small numbers can be recovered much more efficiently than in the case of recovering nucleic acids after having preliminarily isolated the mammalian cells from the stool. By carrying out nucleic acid analysis using nucleic acids recovered in this manner, markers for specific diseases such as colon cancer can be detected with extremely high sensitivity and accuracy. Furthermore, nucleic acids recovered from a stool sample may be DNA, RNA or both DNA and RNA.

For example, after denaturing protein present in the stool sample of the present invention, and eluting nucleic acids from mammalian cells and indigenous intestinal bacteria present in the stool sample in a step (a), by then recovering the eluted nucleic acids in a step (b), nucleic acids derived from mammalian cells and nucleic acids derived from indigenous intestinal bacteria can be simultaneously recovered from the stool sample.

Denaturing of protein present in the stool sample in step (a) can be carried out by a commonly known technique. For example, protein present in the stool sample can be denatured by adding a compound ordinarily used as a protein denaturing agent, such as a chaotropic salt, organic solvent or detergent, to the stool sample. The same compounds listed as examples of chaotropic salts and detergents added to the preparation solution S of the present invention can be used as chaotropic salts and detergents added to the stool sample in step (a). Phenol is a preferable example of an organic solvent. Phenol may be neutral or acidic. In the case of using acidic phenol, RNA can be more selectively extracted into an aqueous layer than DNA. Furthermore, in the case of adding a chaotropic salt, organic solvent or detergent to the stool sample in step (a), one member of compound may be added or two or more members of compounds may be added.

Protein denatured in step (a) may also be removed by providing a step (c) between step (a) and step (b). The quality of recovered nucleic acids can be improved by preliminarily removing denatured protein prior to recovering nucleic acids. A commonly known technique can be used to remove protein in step (c). For example, denatured protein can be removed by precipitating the denatured protein by centrifugal separation and recovering only the supernatant. In addition, centrifugal separation may be carried out after having adding chloroform and adequately agitating and mixing with a vortex and the like to precipitate the denatured protein and recover only the resulting supernatant. As a result, denatured protein can be more completely removed than in the case of simply carrying out centrifugal separation.

Recovery of nucleic acids eluted in step (b) can be carried out using a commonly known technique such as ethanol precipitation or cesium chloride ultracentrifugation. Examples of recovery methods include the following steps (b1) and (b2). Nucleic acids eluted in step (a) are adsorbed to an organic support in step (b1). Subsequently, in step (b2), the nucleic acids adsorbed in step (b1) are eluted from the inorganic support. As a result, the nucleic acids are able to be recovered. A known inorganic support capable of adsorbing nucleic acids can be used for the inorganic support used in step (b1). In addition, there are no particular limitations on the form of this inorganic support, and may be in the form of particles or a film. Examples of this inorganic support include silica-containing particles (beads) such as silica gel, silaceous oxides, glass or diatomaceous earth, and porous films such as Nylon, polycarbonate, polyacrylate or nitrocellulose film.
A solvent normally used to elute nucleic acids from these known organic supports can be suitably used for the solvent used in step (b2) in consideration of the member of nucleic acids recovered, the subsequent nucleic acid analysis method and the like. For example, purified water is particularly preferable for this elution solvent. Furthermore, the inorganic support adsorbed with nucleic acids is preferably washed using a suitable washing buffer after step (b1) and before step (b2).

Furthermore, in the case the stool sample is prepared using the preparation solution S containing a chaotropic salt or detergent at a concentration adequate for eluting nucleic acids from mammalian cells and the like, step (a) can be omitted from the step for recovering nucleic acids from the stool sample.

In the case a stool sample is prepared using a preparation solution S that does not contain an adequate concentration of chaotropic salt or detergent for eluting nucleic acids from mammalian cells and the like, a step (d) is preferably provided before step (a) to recover a solid component from the stool sample. The ratio of liquid component to solid component in the stool is large in order to rapidly mix the stool with the preparation solution S. Therefore, by removing the preparation solution S from the stool sample and recovering only the solid component containing mammalian cells and indigenous intestinal bacteria, the scale of the nucleic acid recovery step and analysis can be reduced. In addition, by removing the water-soluble organic solvent from the solid component, the effect of the water-soluble organic solvent in the step for recovering nucleic acids from the solid component can be inhibited. For example, only the solid component can be recovered by centrifugally separating the stool sample of the present invention and removing the supernatant by precipitating the solid component. In addition, only the solid component can also be recovered by filtering and the like. Moreover, the recovered solid component is preferably washed using a suitable buffer such as phosphate-buffered saline (PBS, pH 7.4).

Furthermore, although a protein denaturing agent such as a chaotropic salt may be added directly to the recovered solid component, the protein denaturing agent is preferably added after first suspending in a suitable elution agent. In the case of recovering DNA, a phosphate buffer or Tris buffer, for example, can be used for this elution agent. An agent in which DNase has been deactivated by high-pressure steam sterilization and the like is preferable, while an agent containing a proteinase such as proteinase K is more preferable. On the other hand, in the case of recovering RNA, although a citrate buffer, for example, can be used for the elution agent, since RNA is a substance that is extremely susceptible to decomposition, it is preferable to use a buffer that contains an RNase inhibitor such as guanidine thiocyanate or guanidine hydrochloride.

In the subsequent analysis method, a buffer into which nucleic acids have eluted after having eluted nucleic acids from mammalian cells or indigenous intestinal bacteria present in a stool sample can also be used directly for nucleic acid analysis without having to recover nucleic acids from the stool sample. For example, in the case a large number of pathogens and the like are present in the stool sample and nucleic acids derived from these pathogens are to be analyzed, only the solid component is recovered from the stool sample followed by adding an elution agent such as PBS containing a proteinase such as proteinase K to obtain a stool sample solution. Genes and the like derived from the pathogens can then be detected by using this resulting stool sample solution directly for nucleic acid analysis. In addition, recovery of nucleic acid from the stool sample can also be carried out using a commercially available kit such as a nucleic acid detection kit or virus detection kit.

Nucleic acids recovered from the stool sample of the present invention can be analyzed using a known nucleic acid analysis method. Examples of nucleic acid analysis methods include methods involving quantification of nucleic acids and methods involving detection of specific base sequence regions using PCR and the like. For example, the presence or absence of the onset of cancer can be investigated by detecting the presence or absence of a base sequence region encoded by a cancer gene or by detecting the presence or absence of a genetic mutation such as a base sequence region containing a microsatellite. In the case of using DNA recovered from a stool sample, analysis of DNA mutations or analysis of epigenetic changes can be carried out. Examples of mutation analyses include analyses of base insertions, deletions, substitutions, duplications or inversions. In addition, examples of analyses of epigenetic changes include analyses of methylation and demethylation. In addition, in the case of recovering RNA, cDNA can be synthesized by a reverse transcription reaction (reverse transcriptase-polymerase chain reaction: RT-PCR) followed by using that cDNA for analysis in the same manner as DNA. In the case of using recovered RNA, mutations such as base insertions, deletions, substitutions, duplications, inversions or splicing variants (isoforms) can be detected in the RNA. In addition, functional RNA (non-coding RNA) analyses such as analyses of transfer RNA (tRNA), ribosomal RNA (rRNA) or microRNA (miRNA) can also be carried out. In addition, the amount of RNA expressed can also be detected. mRNA expression analyses, K-ras gene mutation analyses and DNA methylation analyses are carried out particularly preferably. Furthermore, these analyses can be carried out according to known methods in this field. In addition, a commercially available kit such as a K-ras gene mutation analysis kit or methylation detection kit may also be used.

In this manner, nucleic acids present in stool can be analyzed with high sensitivity and high accuracy by using the stool sample preparation method of the present invention, a method for recovering nucleic acids from a stool sample prepared according to this preparation method, and a nucleic acid analysis method that uses nucleic acids recovered according to this nucleic acid recovery method. Consequently, this can be expected to contribute and be applicable to early detection and diagnosis of various symptoms and diseases, including colon cancer, observation of the course of treatment, and pathological research and the like on other abnormal states.

The present invention enables a collected stool to be prepared more easily and rapidly by collecting the stool in a stool collection container preliminarily containing the preparation solution S. In addition, the effects of the present invention can be demonstrated more readily by using a stool collection kit having the preparation solution S of the present invention and a stool collection container containing that preparation solution S. Furthermore, the stool collection kit may suitably have components such as a stool collection rod in addition to the preparation solution S and the stool collection container containing the preparation solution S.

There are no particular limitations on the shape of size and so on of the stool collection container used in the present invention, and a known stool collection container capable of containing a solvent can be used. A stool collection container in which the cover of the stool collection container and a stool collection rod are integrated into a single unit is preferable since it facilitates handling. In addition, the stool collection rod is more preferably able to collect a fixed amount of stool in order to control the amount of stool collected. An example of such a known stool collection container is the stool collection container disclosed in Japanese Examined Patent Application, Second Publication No. H6-72837.

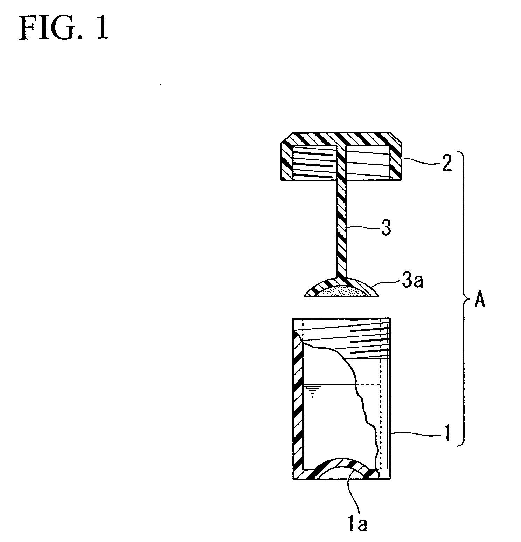

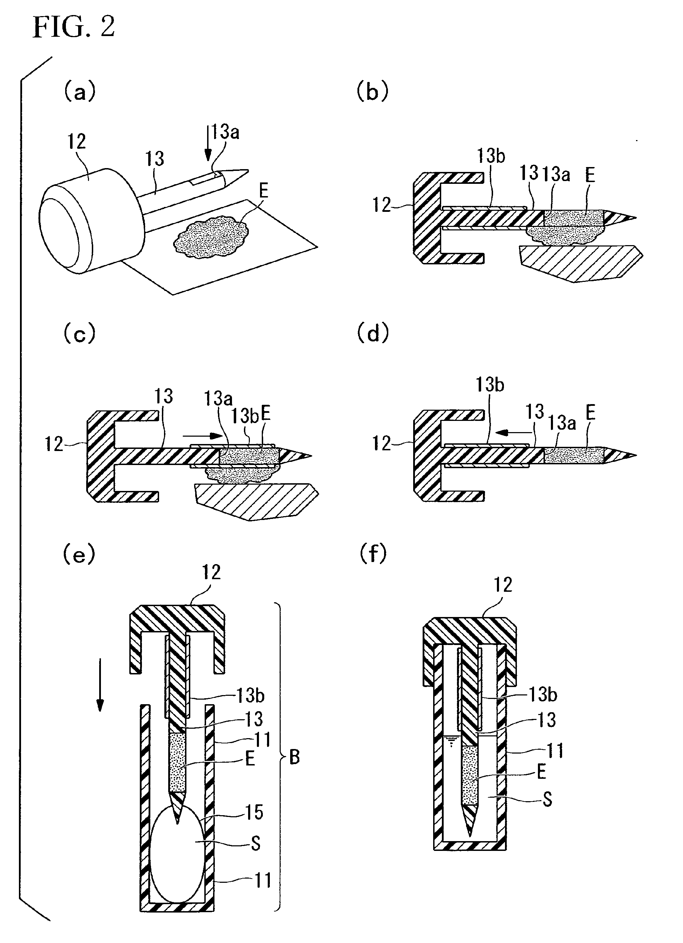

FIGS. 1 and 2 are drawings respectively showing embodiments of stool collection containers A and B able to be used in a stool collection kit of the present invention. Furthermore, stool collection containers able to be used in the stool collection kit of the present invention are not limited to these stool collection containers.

An explanation is first provided of the stool collection container of FIG. 1. The stool collection container A of the present embodiment has a cover 2 integrated with a stool collection rod 3 and a container body 1, and the preparation solution S of the present invention is contained within the container body 1. A cup 3a, capable of collecting a fixed amount of stool, is present on the end of the stool collection rod 3, and a net serving as a sieve is attached to the bottom of this cup 3 a. On the other hand, a protrusion 1a is present on the bottom of the container body 1 that is roughly the same shape as the cup 3a and overlaps with the shape of the cup 3a. Since stool that has been collected in the cup 3 a by engaging the cup 3 a with the protrusion 1a is pushed out from the net attached to the bottom of the cup 3 a, stool can be rapidly dispersed in the preparation solution S.

The stool collection container shown in FIG. 2 is the stool collection container B having a cover 12 integrated into a single unit with a stool collection rod 13 having a tapered end and a container body 11, and has a sealed pouch 15 within the container body 11 that contains the preparation solution S. A slot 13a is formed in the distal end of the stool collection rod 13 that is capable of collecting a fixed amount of a stool E. In addition, a movable cover 13b is attached to both sides of the stool collection rod 13 that covers the slot 13a by sliding over the stool collection rod 13. The following provides an explanation of an embodiment of a usage method of the present stool collection container B.
First, as shown in FIG. 2a, the stool collection rod 13 is pressed against the stool E with the movable cover 13b moved towards the cover 12 rather than the slot 13a so that the slot 13a is completely uncovered. Next, the stool E is filled into the slot 13a as shown in FIG. 2b. While in this state, the movable cover 13b is slid towards the distal end of the stool collection rod 13 to cover the slot 13a thereby separating any excess stool E and making it possible to accurately collect the stool E in an amount equal to the volume of the slot 13a (FIG. 2c). Subsequently, the movable cover 13b is returned to its original position to completely uncover the slot 13a (FIG. 2d) followed by housing the cover 12 in the container body 11 (FIG. 2e). When the stool collection rod 13 is housed in the container body 11, the tapered end of the stool collection rod 13 pierces the pouch 15 containing the preparation solution S, thereby causing the container body 11 to be filled with the preparation solution S to a level equal to or higher than the slot 13a containing the stool E, and enabling the stool E and the preparation solution S to make direct contact (FIG. 2f). At this time, since the container body 11 is sealed by the cover 12, there is no leakage of the preparation solution S. Subsequently, since the container body is moved due to transport and the like, the preparation solution S and stool E within the container body 11 are mixed. Since the stool collection rod 13 is placed in a container and the solution is filled into the container only after this has been carried out in this stool collection container B, even in the case of using a preparation solution S that is harmful to the body in the manner of methanol, accidents caused by leakage of solution can be avoided thereby enabling the stool collection container to be handled safely even in the home.

### [Examples]

Although the following provides a more detailed explanation of the present invention by indicating examples thereof, the present invention is not limited to the following examples. Furthermore, unless specifically indicated otherwise, the term "%" refers to "percent by volume (vol%)". In addition, cultured cells consisting of Caco-2 cells, SW620 cells and MKN45 cells and bacterial cells consisting of Enterobacter aerogenes were cultured in accordance with ordinary methods.

### [Example 1]

0.5 g aliquots of stool collected from a healthy volunteer were dispensed into eight 15 mL polypropylene tubes. 10 mL of the preparation solution S described in Table 1 were added to each stool and dispersed well to respectively prepare stool samples 1-1 to 1-8. Furthermore, preparation solution S supplemented with 0.1 M citric acid was adjusted to the pH values described in Table 1 using NaOH. In addition, the pH of the 60% ethanol solution to which organic acid was not added was the pH prior to adding to stool.

**[Table 1]**

| Preparation Solution | Water-soluble Organic Solvent | Organic Acid | pH |
|---|---|---|---|
| Stool sample 1-1 | 60% ethanol | 0.1 M citric acid | 3.0 |
| Stool sample 1-2 | 60% ethanol | 0.1 M citric acid | 3.5 |
| Stool sample 1-3 | 60% ethanol | 0.1 M citric acid | 4.0 |
| Stool sample 1-4 | 60% ethanol | 0.1 M citric acid | 4.5 |
| Stool sample 1-5 | 60% ethanol | 0.1 M citric acid | 5.0 |
| Stool sample 1-6 | 60% ethanol | 0.1 M citric acid | 5.5 |
| Stool sample 1-7 | 60% ethanol | 0.1 M citric acid | 6.0 |
| Stool sample 1-8 | 60% ethanol | Not added | 6.5 |

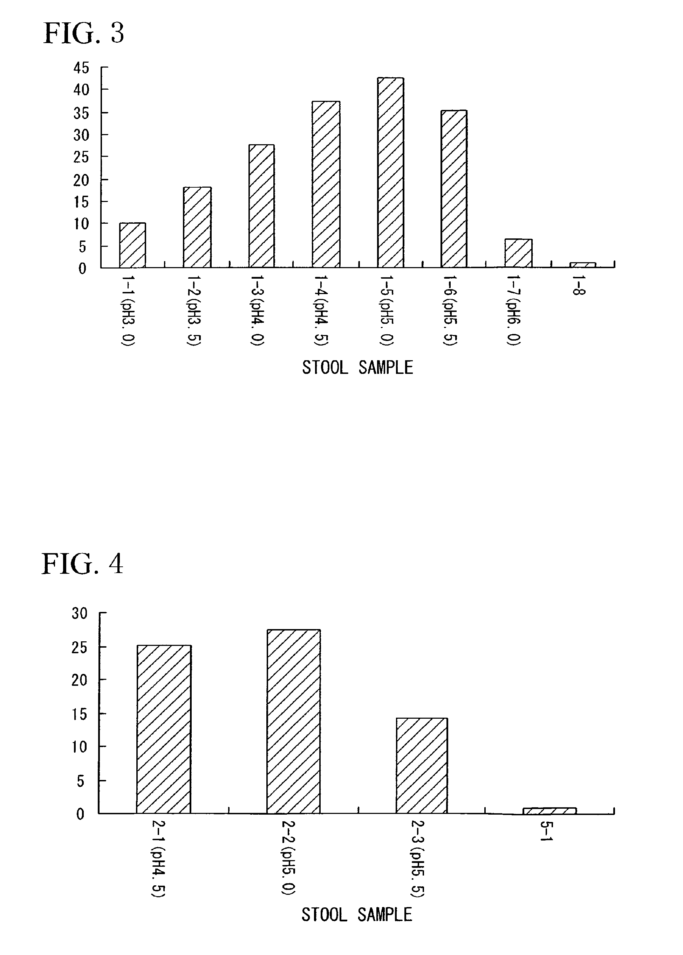

After storing these stool samples for 7 days at 25°C, RNA was recovered from each stool sample. This RNA recovery step consisted of recovering the solid component of the stool by centrifuging each tube followed by adding a phenol mixture known as "Trizol" (Invitrogen) and adequately mixing with a homogenizer. Subsequently, chloroform was added to the mixture of stool sample and Trizol, and after adequately mixing using a vortex, the mixture was centrifuged for 20 minutes at 12,000 x g and 4°C. The supernatant (aqueous layer) was separated by this centrifugation procedure, and this supernatant (aqueous layer) was passed through an RNA recovery column of the RNeasy Midi Kit (Qiagen). RNA was recovered by carrying out a washing procedure and RNA elution procedure on the RNA recovery column of this kit in accordance with the protocol provided.
RT-PCR was carried out on 1 µg of the recovered RNA, and PCR was carried out using the resulting cDNA as a template followed by detection of human GAPDH gene. The GAPDH primer probe MIX (Catalog No.: Hs02786624_gl) was used as primer.
This PCR step consisted of respectively dispensing 1 µL aliquots of the resulting cDNA into a 0.2 mL 96-well PCR plate. Subsequently, 8 µL of ultrapure water and 10 µL of the nucleic acid amplification reagent "TaqMan Gene Expression Master Mix" (Applied Biosystems) were added then to each well followed by respectively adding 1 µL of GAPDH Primer Probe MIX (Applied Biosystems) and mixing to prepare PCR reaction solutions.
PCR was carried out while measuring fluorescence intensity over time by placing this PCR plate in an ABI real-time PCR apparatus, and initially treating for 10 minutes at 95°C followed by carrying out 40 cycles of heat cycling consisting of 1 minute at 95°C, 1 minute at 56.5°C and 1 minute at 72°C, and then further treating for 7 minutes at 72°C. The results of measuring fluorescence intensity were analyzed, and the relative values of the expressed amount of GAPDH gene in the RNA recovered from each sample were calculated.
The results of a relative comparison of the expression levels of GAPDH gene in RNA derived from each stool sample are shown in FIG. 3. On the basis of these results, stool samples 1-1 to 1-7, which used preparation solutions S supplemented with organic acid having buffering action, yielded higher expression levels of GAPDH gene than stool sample 1-8, which used a 60% ethanol solution not having buffering action for the preparation solution S. In addition, results were also obtained indicating that expression levels of GAPDH gene are higher at an acidic pH range of 3 to 6, and particularly 4.5 to 5.5, than at a neutral pH 7. Since stool samples 1-1 to 1-8 consist of RNA recovered from stool derived from a single individual, a high expression level of GAPDH gene indicates a low level of nucleic acid decomposition in the stool and that storage is carried out favorably. Namely, it was determined that a preparation solution S having an acidic pH by adding organic acid demonstrates higher nucleic acid preservation effects than that not supplemented with organic acid.
Furthermore, when the status of ribosomal RNA, which is thought to indicate the degree of nucleic acid decomposition, was confirmed by electrophoresis, two bands were distinctly detected in stool samples 1-3 to 1-6 having pH values of 4.5 to 5.5, thereby confirming decomposition of RNA to be extremely low.

### [Example 2]

Nucleic acid preservation effects were investigated in the case of using adipic acid instead of citric acid for the organic acid added to the preparation solution S. The RNA recovery step and PCR step were carried out in the same manner as Example 1 with the exception of using the preparation solutions S described in Table 2 for the preparation solution S. Furthermore, pH values of the preparation solutions S supplemented with 0.1 M adipic acid were adjusted using NaOH. On the other hand, the pH values of the preparation solutions S supplemented with 0.1 M acetic acid were adjusted using sodium acetate, while the pH values of the preparation solutions S supplemented with 0.1 M lactic acid were adjusted using sodium lactate.

**[Table 2]**

| Preparation Solution | Water-soluble Organic Solvent | Organic Acid | pH I |
|---|---|---|---|
| Stool Sample 2-1 | 60% ethanol | 0.1 M adipic acid | 4.5 |
| Stool Sample 2-2 | 60% ethanol | 0.1 M adipic acid | 5.0 |
| Stool Sample 2-3 | 60% ethanol | 0.1 M adipic acid | 5.5 |
| Stool Sample 3-1 | 60% ethanol | 0.1 M acetic acid | 4.5 |
| Stool Sample 3-2 | 60% ethanol | 0.1 M acetic acid | 5.0 |
| Stool Sample 3-3 | 60% ethanol | 0.1 M acetic acid | 5.5 |
| Stool Sample 4-1 | 60% ethanol | 0.1 M lactic acid | 4.5 |
| Stool Sample 4-2 | 60% ethanol | 0.1 M lactic acid | 5.0 |
| Stool Sample 4-3 | 60% ethanol | 0.1 M lactic acid | 5.5 |
| Stool Sample 5-1 | 60% ethanol | Not added | 6.5 |

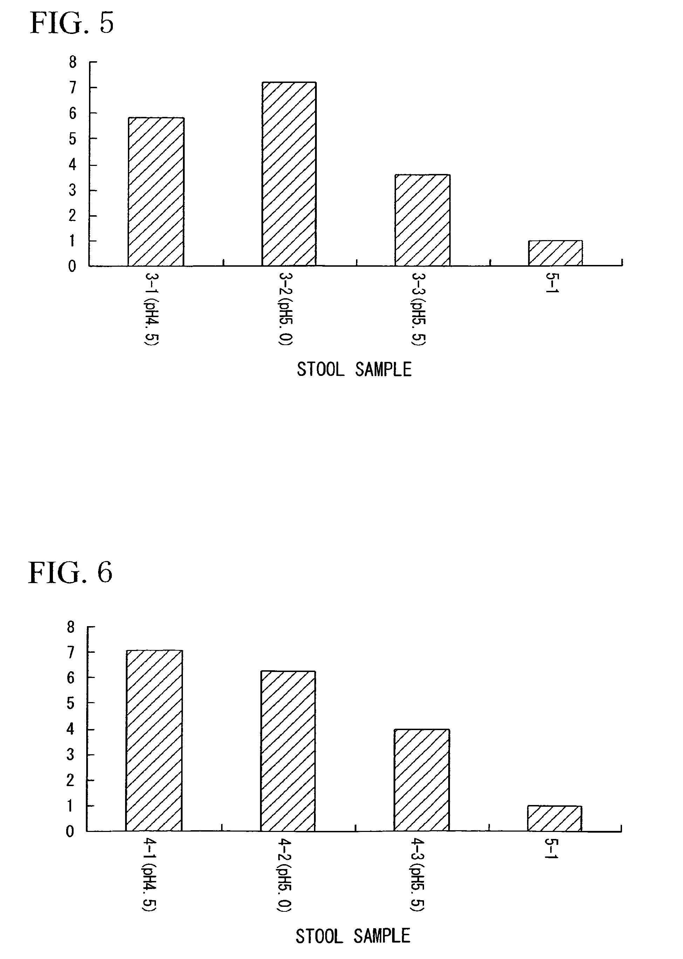

FIG. 4 is a graph showing the results of a relative comparison of stool samples 2-1 to 2-3 in the case of using adipic acid for the organic acid and stool sample 5-1 not supplemented with organic acid. FIG 5 is a graph showing the results of a relative comparison of stool samples 3-1 to 3-3 in the case of using acetic acid for the organic acid and stool sample 5-1 not supplemented with organic acid. FIG. 6 is a graph showing the results of a relative comparison of stool samples 4-1 to 4-3 in the case of using lactic acid for the organic acid and stool sample 5-1 not supplemented with organic acid. As a result of a relative comparison of the expression levels of GAPDH gene in RNA derived from each stool sample, higher nucleic acid preservation effects were determined to be obtained in the case of using any of the organic acids than in the case of stool sample 5-1 not having buffering action as a result of not adding organic acid. In particular, adipic acid was determined to have extremely superior nucleic acid preservation effects in the same manner as citric acid of Example 1. In addition, even in the case of using acetic acid or lactic acid for the organic acid, although nucleic acid preservation effects were slightly inferior to those in the case of using adipic acid or citric acid, expression levels of GAPDH gene were confirmed to be about 7 times higher than that of stool sample 5-1 not supplemented with organic acid, thereby confirming these acids to have favorable nucleic acid preservation effects.
Moreover, when the status of ribosomal RNA was confirmed in the same manner as Example 1, two bands were clearly detected in the RNA derived from all of the stool samples in this example with the exception of stool sample 5-1. On the basis of these results, there was confirmed to be extremely little decomposition of RNA.

### [Reference Example 1]

1 g aliquots of stool collected from a healthy volunteer were dispensed into three 15 mL polypropylene tubes. Freezing treatment was promptly carried out on one of the tubes using liquid nitrogen immediately after dispensing to obtain a stool sample (1A). 10 mL of a 70% ethanol solution were added to another tube after dispensing followed by adequately dispersing the stool and allowing to stand for 1 hour at room temperature to obtain a stool sample (1B). The remaining tube was promptly transferred to an extraction step after dispensing without adding any solution and the like to obtain a stool sample (1C).
Subsequently, RNA was extracted from each stool sample. More specifically, 3 mL of a phenol mixture known as "Trizol" (Invitrogen) were added to each stool sample and adequately mixed for 30 seconds or more with a homogenizer. After adding 3 mL of chloroform to the mixtures and mixing adequately using a vortex, the samples were centrifuged for 20 minutes at 12,000 × g and 4°C. The precipitate (aqueous layer) obtained from this centrifugation was passed through an RNA recovery column of an RNeasy Midi Kit (Qiagen) and RNA was recovered by carrying out a washing procedure and an RNA elution procedure on the RNA recovery column in accordance with the protocol provided. The recovered RNA was quantified using a NanoDrop (NanoDrop).

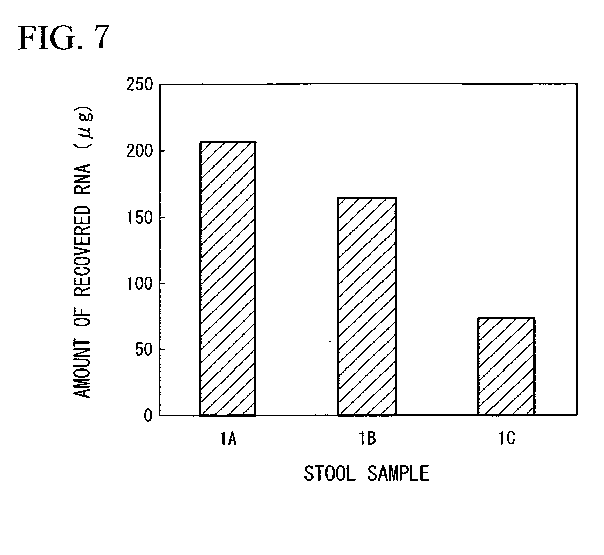

FIG. 7 is a graph showing the amounts of RNA recovered from each stool sample. Although the amount of RNA recovered from the stool sample (1B) prepared using an ethanol solution serving as the preparation solution S was slightly less than the amount of RNA recovered from the stool sample (1A) that was subjected to freezing treatment immediately after dispensing, an extremely large amount of RNA was able to be recovered in comparison with the stool sample (1C) that was subjected to nucleic acid extraction immediately after dispensing without adding any solution and the like. On the basis of these results, preparation of stool samples using the preparation solution S of the present invention was determined to allow the obtaining of stool samples from which nucleic acid can be recovered extremely efficiently even if prepared at room temperature. In the case of a patient collecting stool at home as in the manner of routine health examinations and the like, although it is desirable that the stool sample be able to be prepared at room temperature, the preparation solution S of the present invention is able to adequately respond to this requirement.

### [Reference Example 2]

5.0 × 10⁵ cultured Caco-2 cells derived from human colorectal cancer, which express high levels of MDR1 (multidrug resistance 1) gene, were mixed with 0.5 g of stool from a healthy volunteer to prepare a simulated stool of a colorectal cancer patient. Stool samples were prepared from this simulated colorectal cancer patient stool according to the stool sample preparation method of the present invention.
The preparation method consisted of dispensing 0.5 g aliquots of this simulated colorectal cancer patient stool into 15 mL polypropylene tubes and adding and mixing each of the preparation solutions S shown in Table 3 to prepare stool samples. Furthermore, the "universal collection medium" indicated in the table refers to the storage medium described in Patent Document 4 (containing 500 mL of Pack physiological saline G, 400 mg of sodium bicarbonate, 10 g of BSA, 500 units/L of penicillin G, 500 mg mg/L of streptomycin sulfate, 1.25 mg/L of amphotericin B and 50 mg/L of gentamicin). The prepared stool samples were stored for 1, 3, 7 or 10 days, respectively, in a constant-temperature incubator set to room temperature (25°).

**[Table 3]**

| Stool sample preparation solution | |
|---|---|
| (2A) | 5 ml of 70% methanol solution |
| (2B) | 1 mL of 100% methanol solution |
| (2C) | 5 mL of universal collection medium |
| (2D) | 5 ml of PBS |

Following storage, RNA was recovered from each stool sample and mRNA that is the transcription product of MDR1 gene was attempted to be detected in the recovered RNA. RNA recovery was carried out on the stool sample prepared using the preparation solution S of 2C (namely, universal collection medium) (to be referred to as stool sample (2C)) after first separating mammalian cells containing Caco-2 cells. Nucleic acids derived from mammalian cells and nucleic acids derived from bacteria were simultaneously recovered without separating mammalian cells for the stool samples prepared using a preparation solution S other than the universal collection medium. The method used to separate mammalian cells from stool sample (2C) consisted of adding 5 mL of Histopaque 1077 solution (Sigma) to stool sample (2C) and mixing followed by centrifuging at room temperature for 30 minutes at 200 × g and recovering the layer at the interface between the suspension and the Histopaque 1077 solution (this layer contains mammalian cells). The separated mammalian cells were washed three times with PBS.
Recovery of RNA from the stool samples was carried out in the manner described below. First, 3 mL of a phenol mixture ("Trizol" (Invitrogen)) were added to the stool samples (to the separated mammalian cells only from stool sample (2C)) followed by adequately mixing for 30 seconds or more with a homogenizer. Subsequently, 3 mL of chloroform were added to the mixtures of Trizol and stool sample followed by centrifuging for 10 minutes at 12,000 × g. The supernatants (aqueous layer) obtained from this centrifugation treatment were recovered in new polypropylene tubes. Subsequently, RNA was recovered from the recovered supernatants using an RNeasy Midi Kit (Qiagen).

RT-PCR was carried out on the recovered RNA, and PCR was carried out using the resulting cDNA as template. The primers used consisted of a forward primer for MDR1 gene amplification having the base sequence of SEQ ID NO: 1 and a reverse primer for MDR1 gene amplification having the base sequence of SEQ ID NO: 2.
In this PCR step, 12 µL of ultrapure water and 2 µL of 10× buffer were added to 0.2 mL PCR tubes followed by the addition of 1 µL aliquots each of cDNA, the forward primer of SEQ ID NO:1, the reverse primer of SEQ ID NO: 2, magnesium chloride, dNTP and DNA polymerase and mixing to prepare PCR reaction solutions. PCR reactions were carried out according to reaction conditions consisting of subjecting the PCR tubes to 30 cycles of 30 seconds at 95°C, 30 seconds at 60°C and 1 minute at 72°C. As a result, the resulting PCR products were phoresed using an Agilent DNA1000 LabChip Kit™ (Agilent), and investigating the degree of amplification of the PCR products by measuring the intensity of the resulting bands.

**[Table 4]**

| Storage Period | 1 day | 3 days | 7 days | 10 days |
|---|---|---|---|---|
| Stool sample (2A) | ++ | ++ | ++ | + |
| Stool sample (2B) | ++ | ++ | + | + |
| Stool sample (2C) | - | - | - | - |
| Stool sample (2D) | + | - | - | - |

| | | | | |
|---|---|---|---|---|
| ++: Strong amplification +: Intermediate amplification +/-: Weak amplification -: No amplification | | | | |

Table 4 summarizes the degrees of amplification of the PCR products derived from each stool sample for each storage period. Furthermore, in the table, "stool sample (2A)" indicates a stool sample prepared by using 5 ml of a 70% methanol solution (2A in Table 3) for the preparation solution S, "stool sample (2B)" indicates a stool sample prepared using 1 ml of a 100% methanol solution (2B in Table 3) for the preparation solution S, and "stool sample (2D)" indicates a stool sample prepared using 5 ml of PBS (2D in Table 3) for the preparation solution S.
In stool sample (2D), although amplification of the PCR product was confirmed in the case of a storage period of 1 day, amplification was unable to be confirmed starting at a storage period of 3 days. In contrast, amplification of PCR products was able to be confirmed even after storage for 10 days in the stool samples (2A) and (2B) that were prepared using 2A and 2B for the preparation solution S. On the other hand, in the stool sample (2C) prepared using universal collection medium (2C in Table 3) for the preparation solution S, amplification of PCR product was unable to be confirmed even for a storage period of 1 day.
On the basis of the above results, nucleic acids contained in stool were determined to be able to be recovered efficiently from stool samples prepared according to the preparation method of the present invention. In addition, use of stool samples of the present invention was found to be able to improve the accuracy of RNA analysis. This is presumed to be because use of the preparation solution S of the present invention enables nucleic acids derived from mammalian cells present in stool, and particularly RNA that is susceptible to decomposition, to be stably preserved for as long as the stool is able to be stored for an extended period of time at room temperature.
On the other hand, since amplification of the PCR product derived from stool sample (2C) was not observed, in the case of using a solution containing an antibiotic for the preparation solution S, although bacteria present in stool are eliminated by this antibiotic, the possibility was suggested of decomposition of RNA being promoted due to, for example, the release of RNase from the terminated bacterial cells. In addition, since the number of mammalian cells contained in stool is low, the possibility was also suggested that, in the case of having separated mammalian cells from stool, it may be difficult to recover an adequate amount of nucleic acids in comparison with the nucleic acid recovery method of the present invention in which nucleic acids derived from bacterial cells function as carriers.

### [Reference Example 3]

0, 10, 20, 30, 40, 50, 60, 70, 80, 90 and 100% ethanol solutions were respectively prepared by diluting using ultrapure water. 5 mL aliquots of these ethanol solutions were respectively dispensed into 15 mL polypropylene tubes.
0.5 g aliquots of stool collected from a healthy volunteer were dispensed to each of the tubes followed by allowing to stand undisturbed for 48 hours at 37°C. Subsequently, 3 mL of a phenol mixture, "Trizol" (Invitrogen), were added to the solid component obtained by centrifuging each tube and removing the supernatant followed by mixing well for 30 seconds or more with a homogenizer. Subsequently, 3 mL of chloroform were added to the mixtures of Trizol and stool sample followed by centrifuging for 10 minutes at 12,000 × g. The supernatants (aqueous layer) obtained from this centrifugation treatment were recovered in new polypropylene tubes. Subsequently, RNA was recovered from the recovered supernatants using an RNeasy Midi Kit (Qiagen).

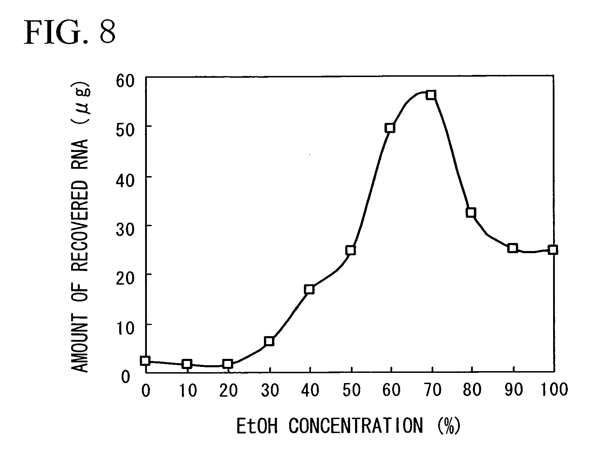

FIG. 8 is a graph showing amounts of RNA recovered from stool samples prepared using each concentration of ethanol solution. On the basis of these results, in the case of using an alcohol such as ethanol as an effective ingredient of the preparation solution S, the alcohol concentration can be seen to preferably be 30% or more, more preferably 50% or more, even more preferably within the range of 50 to 80%, and particularly preferably within the range of 60 to 70%.

### [Reference Example 4]

Stools collected from five healthy volunteers were mixed well, and 0.2 g aliquots were respectively dispensed into two 15 mL polypropylene tubes. After adding 1 mL of a 32% denatured alcohol solution containing 18% isopropanol (50% as total alcohol) to one of the tubes and mixing well, the tube was allowed to stand undisturbed for 1 day at 25°C. This stool sample was designated as stool sample (4A). The remaining tube was used as a control sample, and was recovered in a deep freezer at -80°C immediately after dispensing.
DNA was recovered from both stool samples using a DNA extraction kit ("QIAamp DNA Stool Mini Kit" (Qiagen)). As a result of quantifying the concentrations of the recovered DNA by absorption photometry, nearly equal amounts of DNA were able to be recovered from both stool samples.
Mutation analyses were carried out in accordance with the protocol provided using the "K-ras Codon 12 Mutation Detection Reagent" (Wakunaga Pharmaceutical Co., Ltd.) K-ras gene mutation analysis kit using 100 ng of the recovered DNA. As a result, according to the results of analyzing DNA recovered from the stool sample (4A), all six members of mutant genes were negative in the same manner as the case of using DNA recovered from the control sample.
On the basis of the above results, by using the stool sample production method of the present invention and nucleic acids recovered according to the nucleic acid recovery method of the present invention, nucleic acid analyses can clearly be carried out with favorable accuracy even in the case of nucleic acid analyses requiring high levels of accuracy such as in analyses of gene mutations. In addition, although denatured alcohol consisting of a mixture of isopropanol and ethanol was used for the treatment solution here, similar results were obtained even if using a 50% ethanol solution which has the same alcohol concentration.

### [Reference Example 5]

0.1 g aliquots of tool collected from a healthy volunteer were respectively dispensed into three 15 mL polypropylene tubes, and 3 mL of a 70% ethanol solution was added to one of the tubes followed by adequately dispersing the stool and designating the resulting stool sample as stool sample (5A). On the other hand, 2.4 mL of "ISOGEN" (Nippon Gene) were respectively added to the two remaining tubes followed by adequately dispersing the stool and designating the resulting stool samples as comparative sample (P1) and comparative sample (P2), respectively. Furthermore, "ISOGEN" is a phenol preparation containing 40% phenol (solubility in water of about 10% by weight).
Among these, RNA recovery was carried out on comparative sample (P1) soon after dispersing the stool. This RNA recovery step was carried out by adequately mixing the stool sample for 30 seconds or more with a homogenizer followed by adding 3 mL of chloroform and centrifuging for 10 minutes at 12,000 × g. The supernatant obtained by this centrifugation treatment was recovered in a new polypropylene tube. Subsequently, RNA was recovered from the recovered supernatant using an RNeasy Midi Kit (Qiagen).
In addition, RNA was recovered from comparative sample (P2) in the same manner as comparative sample (P1) after having allowed to stand undisturbed for 5 hours at room temperature.
On the other hand, stool sample (5A) was allowed to stand undisturbed for 5 hours at room temperature in the same manner as comparative sample (P2) followed by removing the supernatant by centrifugal separation. After adding 2.4 mL of "ISOGEN" to the sediment (solid component) obtained by removing the supernatant, RNA recovery was carried out in the same manner as comparative sample (P1).
The recovered RNA was quantified using a NanoDrop (NanoDrop). As a result, although 32 µg of RNA were able to be recovered from comparative sample (P1) for which RNA recovery was carried out immediately after preparing the stool sample, only 14 µg were able to be recovered from comparative sample (P2) for which the recovery procedure was carried out after allowing to stand undisturbed for 5 hours at room temperature. In contrast, the amount of RNA recovered from stool sample (5A) was 57 µg despite having carried out the recovery procedure after allowing to stand undisturbed for 5 hours at room temperature. According to these results, a greater amount of RNA was able to be recovered from stool sample (5A) than comparative sample (P1).
On the basis of these results, use of the stool sample preparation solution of the present invention was determined to allow RNA to be recovered extremely efficiently as compared with the case of using a conventional phenol solution.

### INDUSTRIAL APPLICABILITY

According to the stool sample preparation method of the present invention, since a stool sample that allows nucleic acids in the stool sample to be efficiently preserved can be prepared easily, the present invention can be used particularly in fields such as clinical testing, including routine health examinations, using stool samples.

### BRIEF DESCRIPTION OF THE REFERENCE SYMBOLS

- 1: Container body
- 1a: Protrusion
- 2: Cover
- 3: Stool collection rod
- 3a: Cup
- S: Stool sample preparation solution
- 11: Container body
- 12: Cover
- 13: Stool collection rod
- 13a: Slot
- 13b: Movable cover
- 15: Pouch
- E: Stool

### [SEQUENCE LISTINGS]

PCT International Patent Application No. PCT/JP2009/064789 sequence list

## Claims

1. A stool sample preparation method, comprising: mixing a collected stool with a solution for preparing a stool sample.

2. A solution for preparing a stool sample that is used to mix a collected stool, comprising,
a water-soluble organic solvent containing an organic acid, the water-soluble organic solvent being as an active ingredient of the solution for preparing a stool sample.

3. The solution for preparing a stool sample according to claim 2, wherein the solution for preparing a stool sample has a buffering action.

4. The solution for preparing a stool sample according to claim 2 or claim 3, wherein the water-soluble organic solvent is one or more members selected from the group consisting of a water-soluble alcohol, ketone and aldehyde.

5. The solution for preparing a stool sample according to any of claims 2 to 4, wherein the pH of the solution for preparing the stool sample is 2 to 6.5.

6. The solution for preparing a stool sample according to claim 5, wherein the pH of the solution for preparing the stool sample is 3 to 6.

7. The solution for preparing a stool sample according to any of claims 2 to 6, wherein the organic acid is one or more members selected from the group consisting of a linear aliphatic acid, dicarboxylic acid, amino acid, hydroxy acid and aromatic or heterocyclic polycarboxylic acid.

8. The solution for preparing a stool sample according to claim 7, wherein the organic acid is one or more members selected from the group consisting of acetic acid, lactic acid, citric acid and adipic acid.

9. The solution for preparing a stool sample according to any of claims 6 to 8, wherein the pH of the solution for preparing the stool sample is 4.5 to 5.5.

10. The solution for preparing a stool sample according to any of claims 2 to 9, wherein the organic acid concentration of the solution for preparing the stool sample is 0.01 to 0.1 M.

11. The solution for preparing a stool sample according to any of claims 4 to 10, wherein the water-soluble organic solvent is one or more of a water-soluble alcohol and ketone, and the concentration of the water-soluble organic solvent is 30% or more.

12. The solution for preparing a stool sample according to claim 11, wherein the water-soluble organic solvent contains one or more members selected from the group consisting of ethanol, propanol and methanol as water-soluble alcohol.

13. The solution for preparing a stool sample according to claim 11, wherein the water-soluble organic solvent is ethanol.

14. The solution for preparing a stool sample according to claim 11 or claim 12, wherein the water-soluble organic solvent contains one or more of acetone and methyl ethyl ketone as ketone.

15. The solution for preparing a stool sample according to any of claims 4 to 10, wherein the water-soluble organic solvent is an aldehyde, and the concentration of the water-soluble organic solvent is within the range of 0.01 to 30%.

16. The solution for preparing a stool sample according to any of claims 2 to 15, wherein the mixing ratio of the stool and the solution for preparing the stool sample is such that the ratio of the volume of the solution for preparing the stool sample is 1 or more based on a value of 1 for the volume of the stool.

17. The solution for preparing a stool sample according to any of claims 2 to 16,
wherein the solution for preparing the stool sample contains a detergent.

18. The solution for preparing a stool sample according to any of claims 2 to 17, wherein the solution for preparing the stool sample contains a colorant.

19. The solution for preparing a stool sample according to any of claims 2 to 18, wherein the concentration of the water-soluble organic solvent in the solution for preparing the stool sample is 30% or more.

20. A stool collection kit, comprising:
a solution for preparing a stool sample having a water-soluble organic solvent that contains an organic acid, the water-soluble organic solvent being as an active ingredient the solution for preparing a stool sample and,
a stool collection container containing the solution for preparing a stool sample.

21. The stool collection kit according to claim 20, wherein the concentration of the water-soluble organic solvent in the solution for preparing a stool sample is 30% or more.

22. A stool sample prepared using the solution for preparing a stool sample according to any of claims 2 to 19.

23. A method for recovering nucleic acids from a stool sample, wherein a nucleic acid derived from indigenous intestinal bacteria and a nucleic acid derived from a creature other than indigenous intestinal bacteria are recovered simultaneously from the stool sample according to claim 22.

24. The nucleic acid recovery method according to claim 23, wherein the nucleic acids derived from the creature other than indigenous intestinal bacteria is the nucleic acid derived from a mammalian cell.

25. The nucleic acid recovery method according to claim 23 or claim 24, wherein a step for recovering nucleic acids comprises:
(a) a step for denaturing a protein in the stool sample and eluting the nucleic acid from the indigenous intestinal bacteria and the creature other than indigenous intestinal bacteria in the stool sample, and
(b) a step for recovering the nucleic acid eluted in the step (a).

26. The nucleic acid recovery method according to claim 25, further comprising after the step (a) and before the step (b):
(c) a step for removing the protein denatured by the step (a).

27. The nucleic acid recovery method according to claim 25 or claim 26, wherein denaturation of the protein in the step (a) is carried out using one or more members selected from the group consisting of a chaotropic agent, an organic solvent and a detergent.

28. The nucleic acid recovery method according to claim 27, wherein the organic solvent is phenol.

29. The nucleic acid recovery method according to any of claims 26 to 28, wherein removal of denatured protein in the step (c) is carried out using chloroform.

30. The nucleic acid recovery method according to any of claims 25 to 29, wherein recovery of the nucleic acid in the step (b) further comprising:
(b1) a step for adsorbing the nucleic acid eluted in the step (a) to an inorganic support, and
(b2) a step for eluting the nucleic acids adsorbed in the step (b1) from the inorganic support.

31. The nucleic acid recovery method according to any of claims 25 to 30, having a step (d) before the step (a), the step (d) comprising:
(d) a step for recovering a solid component from the stool sample.

32. A nucleic acid analysis method for analyzing nucleic acid derived from a mammalian cell using the nucleic acid recovered using the nucleic acid recovery method according to any of claims 23 to 31.

33. The nucleic acid analysis method according to claim 32, wherein the mammalian cell is a gastrointestinal cell.

34. The nucleic acid analysis method according to claim 32, wherein the mammalian cell is a large intestine dissociated cell.

35. The nucleic acid analysis method according to any of claims 32 to 34, wherein the nucleic acid derived from the mammalian cell is a marker indicating a neoplastic transformation.

36. The nucleic acid analysis method according to any of claims 32 to 34, wherein the nucleic acid derived from the mammalian cell is a marker indicating an inflammatory gastrointestinal disease.

37. The nucleic acid analysis method according to any of claims 32 to 36, wherein the analysis is one or more of RNA analysis and DNA analysis.

38. The nucleic acid analysis method according to claim 37, wherein the RNA analysis consists of one or more of an analysis of an insertion, deletion, substitution, duplication or inversion of a base in the RNA or a splicing variant, an mRNA expression analysis and a functional RNA analysis.

39. The nucleic acid analysis method according to claim 37, wherein the DNA analysis consists of one or more of a mutation analysis and an analysis of epigenetic changes.

40. The nucleic acid analysis method according to claim 39, wherein the mutation analysis is an analysis of one or more mutations of an insertion, deletion, substitution, duplication or inversion of a base.

41. The nucleic acid analysis method according to claim 39, wherein the analysis is one or more of a DNA methylation analysis and DNA demethylation analysis.

42. The nucleic acid analysis method according to claim 39, wherein the mutation analysis is a mutation analysis of K-ras gene.


SEQUENCE LISTING

<110> OLYMPUS CORPORATION, Ltd.
<120> Method of preparation of stool samples
<130> 09P00917
<160> 2
<170> PatentIn version 3.1

<210> 1
<211> 20
<212> DNA
<213> Artificial Sequence
<220>
<223> Description of Artificial Sequence: primer.
<400> 1
tcattcgagt agcggctctt 20

<210> 2
<211> 20
<212> DNA
<213> Artificial Sequence
<220>
<223> Description of Artificial Sequence: primer.
<400> 2
cttctttgct cctccattgc 20